(19) Europäisches Patentamt
European Patent Office
Office européen des brevets

(11) Publication number: **0 049 126**
**A1**

# (12) EUROPEAN PATENT APPLICATION

(21) Application number: **81304433.6**

(22) Date of filing: **25.09.81**

(51) Int. Cl.³: **C 07 C 39/367**, C 07 C 37/14

(30) Priority: **25.09.80 GB 8031041**

(43) Date of publication of application: **07.04.82**
**Bulletin 82/14**

(84) Designated Contracting States: **AT BE CH DE FR GB IT LI LU NL SE**

(71) Applicant: **COALITE GROUP LIMITED, Buttermilk Lane, Bolsover nr.Chesterfield Derbyshire S44 6AB (GB)**

(72) Inventor: **Gladwin, Keith, 18 Davian Way Walton, Chesterfield Derbyshire (GB)**
Inventor: **Young, Brian Ralph, 4 Eden Street Wingerworth, Chesterfield Derbyshire (GB)**
Inventor: **Gains, Michael David, 36 Valley Road, Spital, Chesterfield Derbyshire (GB)**

(74) Representative: **Seaborn, George Stephen, c/o Edward Evans & Co. Chancery House 53-64 Chancery Lane, London WC2A 1SD (GB)**

## (54) Alpha-methyl-benzyl-chlorophenols and process for their preparation.

(57) The invention provides novel bactericidal compounds, specifically monostyryl ortho- and para-chlorophenols of the general formula

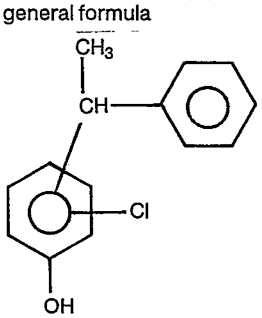

the Cl atom being ortho or para to the OH group.

The compounds of the invention may be prepared by reacting ortho- or para-chlorophenol with styrene. Preferably the reaction is carried out in the presence of an acidic catalyst. Preferably the chlorophenol is in excess relative to the styrene.

EP 0 049 126 A1

TITLE MODIFIED
see front page

0049126

Novel Styryl Halophenols Useful as Bactericides and Process for their Preparation.

The present invention relates to novel compounds useful as bactericides and to a method of preparing such compounds.

In accordance with a first aspect of the present invention, there are provided monostyryl ortho-chlorophenols and monostyryl para-chlorophenols, that is compounds of the general formula

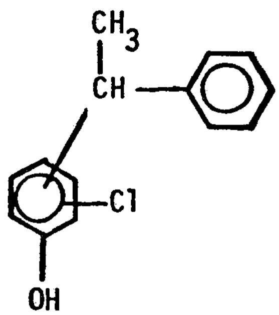

(I) ,

where the Cl atom is ortho or para to the OH group.

There are further provided mixtures of two or more compounds of the general formula (I).

The monostyryl o- and p-chlorophenols according to the invention have good bactericidal properties. In constrast we have found no bactericidal properties exhibited by distyryl chlorophenols and little or no bactericidal properties exhibited by monostyryl meta-chlorophenols and monostyryl methylchlorophenols.

A monostyryl chlorophenol according to the invention may be prepared by a process comprising reacting the corresponding o- or p-chlorophenol with styrene.

In the process of the invention, preferably an excess of the chlorophenol is used to eliminate or reduce production of the corresponding distyryl chlorophenol. Preferably the excess of the chlorophenol is such that the molar ratio of the chlorophenol to the styrene is in the range 2:1 to 3:1.

After the reaction, the excess unreacted chlorophenol may be removed by fractionation to leave a residue comprising monostyryl chlorophenol and, as a by-product, polystyrene. Pure monostyryl chlorophenol can be obtained from the residue by Engler distillation and fractionation. However, such Engler distillation and fractionation might prove expensive and difficult on a large scale due to the low pressure and high temperatures involved.

Preferably the process of the invention is carried out in the presence of an acidic catalyst, more preferably an acidic earth such as acidified Fullers earth.

The following examples are given to illustrate the invention and for comparison. Examples 1 to 5 and 9 to 11 illustrate the invention. Examples 6 to 8 are for comparison.

In the examples, percentages are by weight.

Examples 1 to 8. Preparation of styryl chlorophenols using acidified earth as catalyst

In each of Examples 1 to 8, 2 moles of chlorophenol (e.g.257 g of o-, m- or p-chlorophenol) and 13 g (5%) of Fulcat 22B were heated to 120°C. 83.2g (0.8 moles) of styrene were added dropwise in 10 $cm^3$ aliquots over a period of 30 minutes. (The styrene was added dropwise to minimize production of polystyrene). The reaction mixture was then allowed to cool to 100°C, at which temperature the Fulcat 22B was removed by filtration. The excess chlorophenol was removed by fractionation at a reduced pressure. (Fulcat 22B is acidified Fullers earth).

In Examples 1, 2 and 4 the residue was analysed to determine the amounts of monostyryl chlorophenol and polystyrene contained in it and its absolute Rideal Walker (R.W.) coefficient was determined.

In Examples 3 and 5, 6 and 7 the residue was Engler distilled and fractionated to obtain monostyryl chlorophenol with a purity of about 98% by weight and a residue comprising polystyrene. The Rideal Walker coefficient of the purified product was determined.

The product of Example 8 was tested as a biostat in comparison with an existing commercial bactericidal product, viz. hexa chlorophene (2,2'-dihydroxy-3,3',5,5',6,6'-hexachloro-diphenylmethane). These studies indicated that it was effective against the Gram positive organism S.aureus and ineffective against the Gram negative organism E.coli. It was therefore inferior to the hexachlorophane, which controlled both organisms at comparative concentrations.

Example 9

Preparation of styryl chlorophenol using a Lewis acid catalyst.

257 g.(2 moles) of crude p-chlorophenol (containing o-chlorophenol) reaction mixture containing ½% of aluminium chloride were heated to 120°C and 83.2 g (0.8 moles) of styrene were added dropwise over 4½ hours. The reaction mixture was filtered and the unreacted chloro-phenols removed by fractional distillation. A mixture of styrenated o-chlorophenol and p-chlorophenol was then obtained by Engler distillation of the remaining material at 1 mm Hg. This mixture had a Rideal Walker coefficient (absolute) of 500.

Example 10

Peparation of styryl chlorophenol using a mineral acid catalyst.

257 g. pure p-chlorophenol and 2 ml of concentrated sulphuric acid were heated to 90°C and 83.2 g of styrene were added dropwise over 4½ hours. The reaction mixture was washed with 4% sodium carbonate solution and then with water. The dehydrated reaction mixture contained 23.7% 2-styryl-4-chloro-phenol. Although this is a low figure, it shows that reaction occurs and 2-styryl-4-chloro-phenol can be prepared, in low yield, using concentrated sulphuric acid as catalyst.

Example 11

Preparation of styryl p-chlorophenol using an acidic resin catalyst.

257 g of pure para-chlorophenol and 13 g (5%) of "Amberlyst 15" (as catalyst) were heated to 100°C and 83.2 g. of styrene were added dropwise over 4 hours. The catalyst was removed by filtration and the excess para-chlorophenol was removed by fractional distillation leaving a crude product as residue (154 g). This had a Rideal Walker coefficient (absolute) of 350.

The catalyst was used again in repeating the procedure of the previous paragraph and the crude product (158 g) had a Rideal Walker coefficient (absolute) of 350.

Further details of the examples and results are given in the table below.

Referring to the absolute Rideal Walker coefficients in the table, for comparison it may be noted that the bactericide "Halocide 10" produced by BTP/Cocker has an absolute Rideal Walker coefficient of 200 - 240.

In the examples, where p-chlorophenol was used as the chlorophenol, the resulting styryl chlorophenol is 2-styryl-4-chlorophenol and where p-chlorophenol was used as the chlorophenol, the resulting styryl chlorophenol is mainly 2-chloro-4-styryl-phenol, together with a minor amount of 2-chloro-6-styryl-phenol.

TABLE

| Example | Chlorophenol used | Amount of Styryl Chlorophenol in residue (%) | Amount of Polystyrene in residue (%) | Absolute R.W. coefficient of residue | Absolute R.W. coefficient of purified product |
|---|---|---|---|---|---|
| 1 | Pure P.C.P. | 80.4 | 10 | 364 | |
| 2 | Crude P.C.P | 68.8 | 10 | 333 | |
| 3 | Pure P.C.P. | 74 | | | 550 |
| 4 | Pure O.C.P. | 70 | 10 | 270 | |
| 5 | Pure O.C.P. | 73 | | | 410 |
| 6 | m-chlorophenol (3-chloro-phenol) | | | | 190 |
| 7 | 4-chloro-2-methyl-phenol | | | | less than 150 |
| 8 | 6-chloro-2-methyl-phenol | 2 | | See above regarding bactericidal activity | |
| 9 | Crude P.C.P. | | | | 500 |
| 10 | Pure P.C.P. | 23.7 | | | |
| 11 | Pure P.C.P. | | | 350 | |

P.C.P. = p-chlorophenol (4-chlorophenol)
O.C.P. = o-chlorophenol (2-chlorophenol)
Crude P.C.P. contains approx.20% O.C.P.
Percentages are by weight.

CLAIMS

1. A monostyryl ortho- or para-chlorophenol of the general formula:

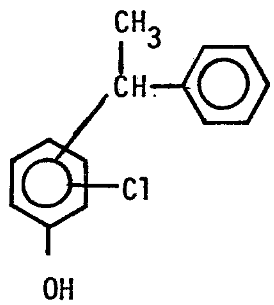

the Cl atom being ortho- or para to the OH group.

2. A mixture of two or more compounds as claimed in claim 1.

3. 2-styryl-4-chlorophenol of the formula

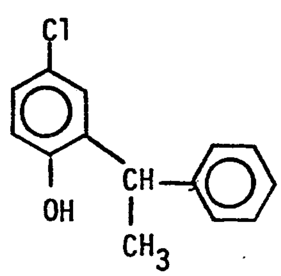

4. 2-chloro-4-styryl-phenol of the formula

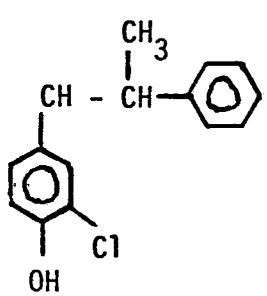

5. A process of preparing a compound according to claim 1, comprising reacting ortho- or para-chlorophenol with styrene.

6. A process according to claim 5, wherein the reaction is carried out in the presence of an acidic catalyst.

7. A process according to claim 6, wherein the catalyst is an acidified earth.

8. A process according to any of claims 5 to 7, wherein the chlorophenol is present in excess relative to the styrene in carrying out the reaction.

9. A process according to claim 8, wherein the molar ratio of the chlorophenol to the styrene is in the range 2:1 to 3:1.

European Patent Office

# EUROPEAN SEARCH REPORT

0049126

Application number
EP 81 30 4433

| Category | DOCUMENTS CONSIDERED TO BE RELEVANT<br>Citation of document with indication, where appropriate, of relevant passages | Relevant to claim | CLASSIFICATION OF THE APPLICATION (Int. Cl.3) |
|---|---|---|---|
| X | JOURNAL OF THE AMERICAN CHEMICAL SOCIETY, vol. 76, no. 18, September 20, 1954, The American Chemical Society WASHINGTON, D.C. (US) H. HART et al.:"Stereochemical Evidence for a Concerted Displacement Mechanism in Acidic Aromatic Alkylations. The Nuclear Alkylation of Phenols with α-Phenethyl Chloride" pages 4547-4550<br>* page 4548, left-hand column, scheme; page 4549, right-hand column, paragraph 5 *<br>-- | 1,3 | C 07 C 39/367<br>37/14 |
| X | US - A - 2 247 402 (R.P. PERKINS et al.)<br>* page 1, right-hand column, lines 3-34; example 3; claims; page 1,left-hand column, lines 36-47 *<br>-- | 1,3-6, 8,9 | TECHNICAL FIELDS SEARCHED (Int. Cl.3)<br>C 07 C 39/367<br>37/14 |
| | US - A - 3 800 051 (J.W. BARNHART)<br>* claims *<br>-- | 1 | |
| A | FR - A - 2 346 312 (VEB JENAPHARM) | 1-9 | CATEGORY OF CITED DOCUMENTS<br>X: particularly relevant<br>A: technological background<br>O: non-written disclosure<br>P: intermediate document<br>T: theory or principle underlying the invention<br>E: conflicting application<br>D: document cited in the application<br>L: citation for other reasons |
| A | US - A - 2 010 595 (E. KLARMANN)<br>------ | 1 | |
| X | The present search report has been drawn up for all claims | | &: member of the same patent family, corresponding document |

| Place of search | Date of completion of the search | Examiner |
|---|---|---|
| The Hague | 26-11-1981 | DELHOMME |

EPO Form 1503.1 06.78